# EUROPÄISCHE PATENTANMELDUNG

(11) **EP 2 436 331 A1**
(43) Veröffentlichungstag der Anmeldung: **04.04.2012**
(21) Anmeldenummer: 11182538.6
(22) Anmeldetag: 23.09.2011
(51) Int. Cl.: A61B 18/18, A61N 1/06, A61N 1/40

(54) **Elektromedizinische Vorrichtung zum nicht-invasiven Reduzieren oder Entfernen von subkutanem Fettgewebe**

(30) Priorität: 29.09.2010 DE 102010041649
(71) Anmelder: Zimmer MedizinSysteme GmbH, 89231 Neu-Ulm (DE)
(72) Erfinder: Bussek, Karlheinz, 86459 Deubach (DE)
(74) Vertreter: Hassa, Oliver Michael

(57) **Zusammenfassung**

Elektromedizinische Vorrichtung zum nicht-invasiven Reduzieren oder Entfernen von subkutanem Fettgewebe,

- mit einer Energiequelle, die einen hochfrequenten Wechselstrom bereitstellt,
- mit zumindest zwei Einzelstrahlern, die von der Energiequelle gespeist werden und die dazu ausgelegt sind, hochfrequente elektromagnetische Wellen in subkutanes Fettgewebe auszustrahlen, und
- mit einer Richtwirkungs-Steuerung, die mit den Einzelstrahlern gekoppelt ist und die die Einzelstrahler derart ansteuert, dass durch Richtungs- und Feldbündelung der von den jeweiligen Einzelstrahlern ausgesendeten hochfrequenten elektromagnetischen Wellen ein elektromagnetisches Gesamtfeld mit einer gewünschten Feldgeometrie in dem subkutanen Fettgewebe erzeugbar ist.

## Beschreibung

Die Erfindung betrifft eine elektromedizinische Vorrichtung zum nicht-invasiven Reduzieren oder Entfernen von subkutanem Fettgewebe.

Überschüssiges Fettgewebe wird bei übergewichtigen Personen heute meist operativ entfernt, beispielsweise durch Fettabsaugen (Liposuktion).Dabei werden Fettzellen der ausgewählten Stellen unter der Haut mittels Hohlnadeln abgesaugt. Solche invasiven Therapieverfahren der plastischen Chirurgie bergen aber immer ein gewisses Risiko, sofern Komplikationen bei der Operation und bei der anschließenden Heilung vorkommen.

Die US 5,143,063 beschreibt ein Verfahren zur Entfernung von fetthaltigem Gewebe mittels eines Fokussierens von Mikrowellen auf das zu entfernende Fettgewebe. Zur Feldbündelung wird dort eine Fokussiereinrichtung in Form eines Parabolspiegels verwendet. Die Verwendung von Parabolspiegeln als Einzelstrahler erfordert einen sehr material- und kostenintensiven Produktionsweg für die elektromedizinische Vorrichtung, insbesondere wenn die Vorrichtung mehrere Einzelstrahler aufweisen soll. Ferner weist das Fokussier- und Bündelungsvermögen der beschrieben Apparatur durch die Verwendung von Parabolspiegeln als Fokussiereinrichtung Grenzen auf, die den Erfordernissen, wie sie bei einer medizinischen Anwendung gewünscht sind, nicht entsprechen.

Die Druckschrift US 5,507,790 beschreibt ein Verfahren zur nicht-invasiven Entfernung von Fettgewebe. Für die Bestrahlung von zu entfernendem Fettgewebe werden dort Mikrowellen-linsen zur Fokussierung eingesetzt. Zusätzlich zu dieser, auf einer komplexen Mikrowellenoptik beruhenden, Apparatur kommen auch Medikamente mit einer metabolischen Wirkung zum Einsatz, die einen erhöhten Fettstoffwechsel generieren sollen. Dabei wird eine Volumenreduzierung der Fettzellen erreicht, ohne einen Zelltod der Fettzellen zu bewirken. Die verwendete komplexe Mikrowellenoptik weist Mikrowellen-Linsen auf, welche bekannterweise eine geringe Güte und einen hohe Abbildungsfehler aufweisen. Diese Eigenschaften behindern eine vorteilhafte Fokussierung der verwendeten Mikrowellenstrahlung im Sinne einer effektiven Behandlung, da eine Beschränkung der Bestrahlungsleistung auf den gewünschten Behandlungsbereich nur schwer zu realisieren ist.

Die der vorliegenden Erfindung zugrunde liegende Aufgabe besteht darin, auf einfach Weise überschüssiges subkutanes Fettgewebe nicht-invasiv zu entfernen oder zu reduzieren.

Erfindungsgemäß wird diese Aufgabe durch eine elektromedizinische Vorrichtung mit den Merkmalen des Patentanspruchs 1 gelöst.

Demgemäß ist vorgesehen:
Eine elektromedizinische Vorrichtung zum nicht-invasiven Reduzieren oder Entfernen von subkutanem Fettgewebe, mit einer Energiequelle, die einen hochfrequenten Wechselstrom bereitstellt, mit zumindest zwei Einzelstrahlern, die von der Energiequelle gespeist sind und die dazu ausgelegt sind, hochfrequente elektromagnetische Wellen in subkutanes Fettgewebe auszustrahlen, und mit einer Richtwirkungs-Steuerung, die mit den Einzelstrahlern gekoppelten ist und die die Einzelstrahler derart ansteuert, dass durch Richtungs- und Feldbündelung der von den jeweiligen Einzelstrahlern ausgesendeten,
   hochfrequenten elektromagnetischen Wellen ein elektromagnetisches Gesamtfeld mit einer gewünschten Feldgeometrie in dem subkutanen Fettgewebe erzeugbar ist.

Die der Erfindung zugrunde liegende Idee besteht darin, die einzelnen Einzelstrahler der erfindungsgemäßen elektromedizinischen Vorrichtung auf einfache Weise derart anzusteuern, dass eine bestmögliche Anpassung des elektromagnetischen Gesamtfeldes an die Körperformen der zu behandelnden Personen erfolgen kann. Aufwändige mechanische Einstellvorgänge der elektromedizinischen Vorrichtung sind vorteilhafterweise entbehrlich.

Eine kontrollierte und gezielte lokale Gewebeerwärmung führt zu dem gewünschten Reduzieren oder Entfernen von subkutanem Fettgewebe.

Vorteilhafte Ausgestaltungen und Weiterbildungen sind Gegenstand der weiteren Unteransprüche sowie der Beschreibung in Verbindung mit den Figuren der Zeichnung.

Gemäß einer bevorzugten Weiterbildung weist die elektromedizinische Vorrichtung im Wesentlichen matrixförmig und/oder kaskadenförmig angeordnete Einzelstrahler auf. Bei einer kaskadenförmigen Anordnung der Einzelstrahler ist eine Hintereinanderschaltung oder ein nacheinander Verketten der Einzelstrahler vorgesehen. Bei einer matrixförmigen Anordnung der Einzelstrahler sind die Einzelstrahler arraymäßig, also in Spalten und Zeilen zueinander angeordnet und miteinander verbunden.

Mittels der matrixförmigen bzw. kaskadenförmigen Anordnung der Einzelstrahler ist vorzugsweise die Erzeugung einer Vielzahl verschiedenartig ausgestalteter Feldgeometrien in einfacher Weise möglich. Ferner ist ein prompter und bedarfsgemäßer Wechsel zwischen diesen Feldgeometrien durch eine einfache und angepasste Ansteuerung der Einzelstrahler der elektromedizinischen Vorrichtung möglich.

Gemäß einer weiteren bevorzugten Ausgestaltung ist mindestens einer der Einzelstrahler als Dipolantenne ausgebildet. Durch die Ausgestaltung des Einzelstrahlers als Dipol kann eine gewünschte Abstrahlgeometrie des Einzelstrahlers bewirkt werden.

Gemäß einem weiteren bevorzugten Ausführungsbeispiel ist mindestens eine der Dipolantennen als λ/2-Dipol oder λ-Dipol ausgebildet. Dadurch können von der Vielzahl der Einzelstrahler der erfindungsgemäßen Vorrichtung jeweils unterschiedliche Einzelgeometrien des elektrischen Feldes erzeugt werden.

Gemäß einem weiteren bevorzugten Ausführungsbeispiel ist mindestens einer der Einzelstrahler als Punktstrahler ausgebildet. Dadurch kann ein besonders einfacher und kostengünstiger Aufbau der elektromedizinischen Vorrichtung realisiert werden.

Gemäß einem weiteren bevorzugten Ausführungsbeispiel sind die jeweiligen Einzelstrahler derart ausgerichtet, dass die Einzelstrahler die von ihnen erzeugten elektromagnetischen Wellen jeweils in dieselbe Richtung ausstrahlen. Insbesondere kann so sichergestellt werden, dass die Einzelstrahler das ausgestrahlte elektromagnetische Feld in die Richtung des Körpers einer zu behandelnden Person ausstrahlt. Dieselbe Richtung ist damit dadurch vergeben, wie und auf welche Weise die Person von der erfindungsgemäßen Vorrichtung appliziert wird, d. h. an die Person angelegt wird. Die Richtung dieses Anlegens der Vorrichtung bestimmt damit auch die vorgegebene Richtung.

Gemäß einem weiteren bevorzugten Ausführungsbeispiel ist die Steuereinrichtung dazu ausgelegt, die Frequenzen und/oder die Amplitude und/oder die Leistung der ausgestrahlten elektromagnetischen Wellen eines Einzelstrahlers zu verändern.

Wie bereits oben ausgeführt, wird durch die erfindungsgemäße Vorrichtung das elektromagnetische Feld variiert. Dabei wird das elektromagnetische Feld insbesondere im Hinblick auf seine Intensität und Ausdehnung sowie seine Homogenität verändert. Diese Veränderung lässt sich auf einfache Weise über eine Variation der Frequenz, der Amplitude und/oder der Leistung der ausgestrahlten elektromagnetischen Wellen des Einzelstrahlers verändern. Auf diese Weise kann je nachdem in welcher Tiefe das subkutane Fettgewebe im Körper einer zu behandelnden Person vorhanden ist sowie welche Dichte und welche Konsistenz dieses Fettgewebe aufweist, das elektromagnetische Feld eben genau angepasst werden.

Gemäß einem weiteren bevorzugten Ausführungsbeispiel weist die Steuereinrichtungen zumindest ein Laufzeitglied auf. Ein jeweiliges Laufzeitglied ist dazu eingerichtet, das von einem Einzelstrahler erzeugte elektromagnetische Feld durch eine Änderung einer jeweiligen Signallaufzeit des Steuersignals zu verändern. Durch Variation und geeignete Anpassung der Signallaufzeiten der verschiedenen Einzelstrahler lässt sich so das elektromagnetische Gesamtfeld bedarfsmäßig einstellen.

Gemäß einem weiteren bevorzugten Ausführungsbeispiel weist ein Steuerglied zumindest einen Phasenschieber auf. Ein jeweiliger Phasenschieber ist dazu eingerichtet, das jeweilige elektromagnetische Feld der Einzelstrahler durch eine Änderung seiner jeweiligen Phase zu verändern. Auch durch diese Maßnahme lässt sich eine bedarfsmäßige Geometrieänderung des elektromagnetischen Gesamtfeldes bewirken.

Gemäß einem weiteren bevorzugten Ausführungsbeispiel ist die Vorrichtung dazu ausgelegt ist, im Frequenzbereich zwischen 0,4-61,5 GHz, vorzugsweise zwischen 1,0-7,5 GHz, und besonders bevorzugt zwischen 1,6-5,9 GHz, und insbesondere zwischen 2,4-2,5 GHz zu arbeiten. Durch die Verwendungen unterschiedlicher Frequenzbereiche kann im Sinne der Fettentfernung oder Fettreduzierung eine solche Strahlung verwendet werden, die ein bestmögliches Absorptionsverhalten der Strahlung im zu behandelnden Bereich erzielt.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird der Fachmann auch Einzelaspekte als Verbesserungen oder Ergänzungen zu der jeweiligen Grundform der vorliegenden Erfindung hinzufügen.

### Zusammenfassung der Figuren

Die vorliegende Erfindung wird nachfolgend anhand der in den schematischen Figuren der Zeichnungen angegebenen Ausführungsbeispiele näher erläutert. Dabei zeigen:
Fig. 1a eine schematische Darstellung eines Ausführungsbeispiels einer erfindungsgemäßen Vorrichtung;
Fig. 1b eine schematische Darstellung eines weiteren Ausführungsbeispiels einer erfindungsgemäßen Vorrichtung;
Fig. 2 die Abstrahlcharakteristik eines λ/2-Dipols in einem Polardiagramm, wie es mittels einer erfindungsgemäßen Vorrichtung erzeugt wird;
Fig. 3 die Abstrahlcharakteristik eines λ-Dipols in einem Polardiagramm, wie es mittels einer erfindungsgemäßen Vorrichtung erzeugt wird;
Fig. 4 die Abstrahlcharakteristik eines 3λ/2-Dipols in einem Polardiagramm, wie es mittels einer erfindungsgemäßen Vorrichtung erzeugt wird;
Fig. 5 eine Darstellung eines menschlichen Gewebebereichs und seine räumliche Lage zu einem elektromagnetischen Gesamtfeld, welches von drei Dipolantennen der erfindungsgemäßen Vorrichtung erzeugt wird.

In den Figuren bezeichnen dieselben Bezugszeichen gleiche oder funktionsgleiche Elemente, Signale und Komponenten - sofern nichts Gegenteiliges angegeben ist.

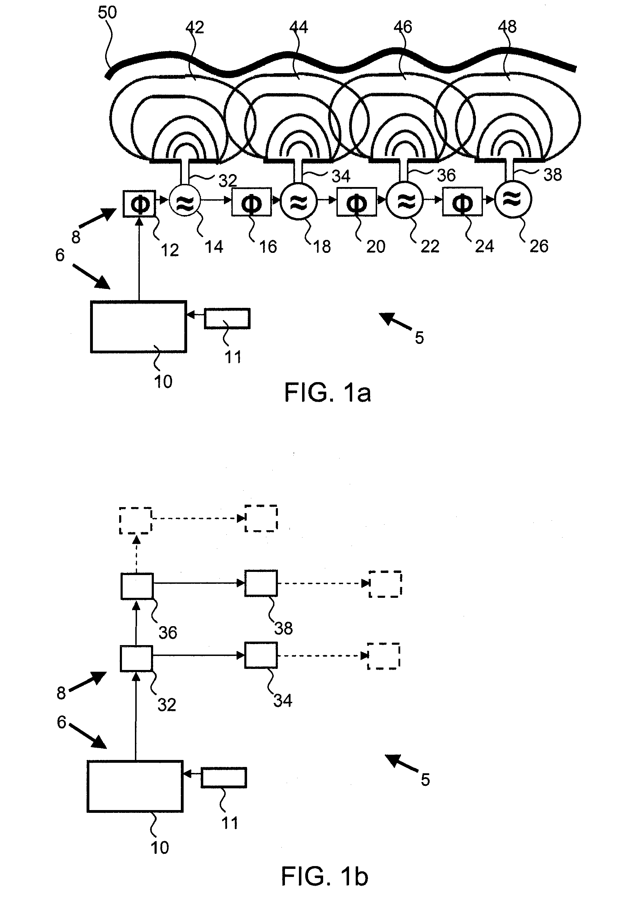

Fig. 1a zeigt eine schematische Darstellung des Aufbaus eines Ausführungsbeispiels einer elektromedizinischen Vorrichtung 5 zum nicht-invasiven Reduzieren oder Entfernen von subkutanem Fettgewebe, gemäß einer ersten Ausführungsform der vorliegenden Erfindung.

Die elektromedizinische Vorrichtung 5 umfasst einen Basisteil 6, welcher eine Richtwirkungs-Steuerung 10 und eine Energiequelle 11 umfasst. Ferner umfasst die elektromedizinische Vorrichtung 5 einen Erweiterungsteil 8, welcher mit variabler Zusammensetzung seines beispielsweise kaskaden- oder matrixförmig ausgestalteten Aufbaus aus weiteren Untereinrichtungen zusammengesetzt.

Die Richtwirkungs-Steuerung 10 ist zum einen mit der Energiequelle 11 elektrisch verbunden und zum anderen beispielsweise mit einem ersten Steuerglied 12. Das erste Steuerglied 12 ist ferner über eine erste Steuereinrichtung 14 an einen ersten Einzelstrahler 32 gekoppelt. Ferner ist an der erste Steuereinrichtung 14 auch ein zweites Steuerglied 16 angeschlossen, welches wiederum mit einer zweiten Steuereinrichtung 18 gekoppelt ist. Die zweite Steuereinrichtung 18 besitzt neben dieser Verbindung zum einen eine Verbindung mit einem zweiten Einzelstrahler 34 und zum anderen mit einem dritten Steuerglied 20. Das dritte Steuerglied 20 ist ferner mit einer dritten Steuereinrichtung 22 verbunden und die dritte Steuereinrichtung 22 ist darüber hinaus mit einem dritten Einzelstrahler 36 und mit einem vierten Steuerglied 24 gekoppelt. Das vierte Steuerglied 24 ist dann wiederum mit einer vierten Steuereinrichtung 26 verbunden. Die vierte Steuereinrichtung 26 ist mit einem vierten Einzelstrahler 38 verbunden. In Fig. 1 sind beispielhaft vier Einzelstrahler mit entprechenden Steuereinrichtungen und Steuergliedern gezeigt - es versteht sich jedoch, dass auch jede andere Vielzahl von Einzelstrahlern möglich ist.

Die Energiequelle 11 versorgt über die Richtwirkungs-Steuerung 10 die elektromedizinische Vorrichtung 5 mit Energie. Die Richtwirkungs-Steuerung 10 speist ferner ein hochfrequentes Signal in das erste Steuerglied 12 ein. Das erste Steuerglied 12 speist dann ein hochfrequentes Signal in die erste Steuereinrichtung 14 ein, in welcher das hochfrequente Signal geteilt wird. Ein erster Teil des hochfrequenten Signals ist zur Abstrahlung über den ersten Einzelstrahler 32 vorgesehen. Ein zweiter Teil des hochfrequenten Signals wird von der ersten Steuereinrichtung 14 über das zweite Steuerglied 16 in die zweite Steuereinrichtung 18 eingespeist, in welcher wiederum eine Teilung in einen zur Abstrahlung über den zweiten Einzelstrahler 34 vorgesehenen Teil und in einen zur Weiterleitung zu dem dirtten Steuerglied 20 vorgesehen Teil erfolgt. Das dritte Steuerglied 20 speist das hochfrequente Signal weiter in die dritte Steuereinrichtung 22 ein. Die dritte Steuereinrichtung speist ein Signal zur Abstrahlung in den dritten Einzelstrahler 36 ein und leitet ferner ein Signal zu dem vierten Steuerglied 24 weiter. Das vierte Steuerglied 24 versorgt die vierte Steuereinrichtung 26, welche den vierten Einzelstrahler 38 zur Abstrahlung speist.

Durch die vorliegende Kaskadierung der Steuerglieder 12, 16, 20, 24 und der Steuereinrichtungen 14, 18, 22, 26 wird jeder Einzelstrahler 32, 34, 36, 38 einzeln mit einem steuerbaren und insbesondere in der Phase und Frequenz variablen, hochfrequenten Signal versorgt. Diese Kaskadierung von Steuergliedern und Steuereinrichtungen im Zusammenspiel mit der Richtwirkungs-Steuerung 10 ermöglicht die erfindungsgemäße Ausbildung einer gewünschten Feldgeometrie des elektromagnetischen Gesamtfeldes 50, welches durch Überlagerung der von den jeweiligen Einzelstrahlern 32, 34, 36, 38 abgestrahlten elektromagnetischen Felder 42, 44, 46, 48 entsteht.

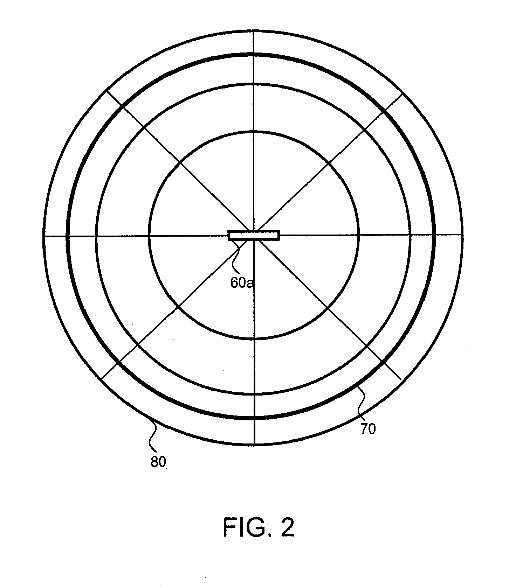

Die Einzelstrahler 32, 34, 36, 38 können dabei jeweils als Dipolantenne ausgebildet sein, insbesondere als λ/2-Dipolantenne, als λ-Dipolantenne oder als 3λ/2-Dipolantenne. Die Abstrahlcharakteristiken derartiger beispielhafter Antennenformen für die Einzelstrahler werden weiter unten in Bezug auf die Fig. 2 bis 4 näher erläutert.

Das erste Steuerglied 12 kann beispielsweise ein Laufzeitglied und/oder einen Phasenschieber umfassen, wobei das Laufzeitglied zur Laufzeitänderung des eingespeisten hochfrequenten Signals bestimmt ist und der Phasenschieber zur Phasenänderung des eingespeisten hochfrequenten Signals ausgelegt ist. Analoges gilt für die Steuerglieder 16, 20 und 24.

Die Steuereinrichtungen 14, 18, 22, 26 sind mit den Einzelstrahlern 32, 34, 36, 38 gekoppelt. Ferner sind die Steuereinrichtungen 14, 18, 22, 26 vorzugsweise dazu bestimmt, die an die Einzelstrahler eingespeisten hochfrequenten Signale bezüglich ihrer Frequenz und/oder Leistung ändern zu können. Fig. 1b zeigt eine schematische Darstellung einer elektromedizinischen Vorrichtung gemäß einer weiteren Ausführungsform der Erfindung. Die elektromedizinische Vorrichtung 5 umfasst dabei ebenso wie in Fig. 1a eine Energiequelle 11 und eine Richtwirkungs-Steuerung 10, die in einem Basisteil 6 angeordnet sein können. Weiterhin umfasst die elektromedizinische Vorrichtung 5 Einzelstrahler 32, 34, 36, 38, die mit der Richtwirkungs-Steuerung 10 gekoppelt sind und von der Energiequelle 11 mit Energie versorgt werden. Wie in Fig. 1b gezeigt, sind die Einzelstrahler 32 und 34 und die Einzelstrahler 36 und 38 jeweils kaskadenartig in einer horizontalen Anordnung hintereinander gekoppelt. Dabei sind die Kaskaden der Einzelstrahler 32 und 34 sowie der Einzelstrahler 36 und 38 vertikal übereinander in einer Matrix angeordnet. Die vorliegende Anordnung der Einzelstrahler 32, 34, 36 und 38 ist nur beispielhafter Natur. Selbstverständlich sind andere Kaskaden- und Matrixanordnung, beispielsweise mit versetzt übereinander angeordneten Kaskadenreihen oder mit Kaskadenreihen, die eine unterschiedliche Anzahl von Einzelstrahlern aufweisen, möglich. In Fig. 1b sind daher zur Veranschaulichung weitere Einzelstrahler gestrichelt angedeutet, die zusätzlich zu den Einzelstrahlern 32, 34, 36 und 38 vorgesehen werden können. Die Anzahl der Einzelstrahlen ist in Fig. 1b mit vier angegeben, jedoch kann eine beliebige Anzahl von Einzelstrahlern ab einer Anzahl von zwei gleichermaßen verwendet werden.

Fig. 2 zeigt ein schematisches Diagramm der Abstrahlcharakteristik eines λ/2-Dipols in einem Polardiagramm, gemäß einer ersten Ausführungsform der vorliegenden Erfindung.

Die in einem Polarkoordinatensystem 80 aufgetragene Abstrahlcharakteristik eines λ/2-Dipols weist eine Kugelcharakteristik 70 auf und hängt von den Abmessungen des Einzelstrahlers im Verhältnis zur Wellenlänge der abgestrahlten Strahlung ab und ihre Formung kann über eine Veränderung der Frequenz eingestellt werden. Im Fall des λ/2-Dipols 60a ist keine Richtwirkung der Abstrahlcharakteristik gegeben.

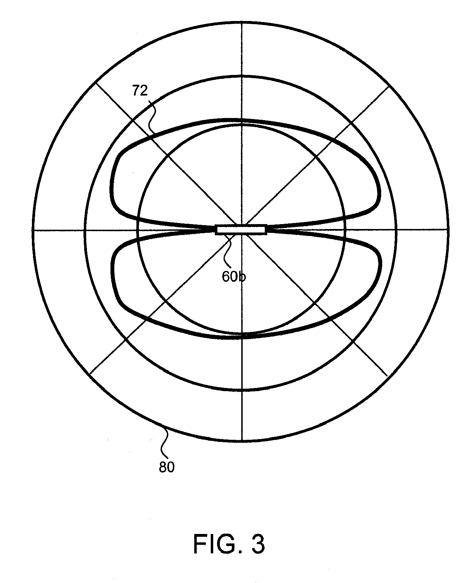

Fig. 3 zeigt ein schematisches Diagramm der Abstrahlcharakteristik eines λ-Dipols in einem Polardiagramm, gemäß einer ersten Ausführungsform der vorliegenden Erfindung.

Die in einem Polarkoordinatensystem 80 aufgezeigte Abstrahlcharakteristik eines λ-Dipols weist eine Achtercharakteristik 72 auf und hängt von den Abmessungen des Einzelstrahlers im Verhältnis zur Wellenlänge der abgestrahlten Strahlung ab und ihre Formung kann über eine Veränderung der Frequenz eingestellt werden. Im Fall des λ-Dipols 60b ist eine Richtwirkung der Abstrahlcharakteristik mit Abstrahlmaxima senkrecht zur Dipolachse gegeben.

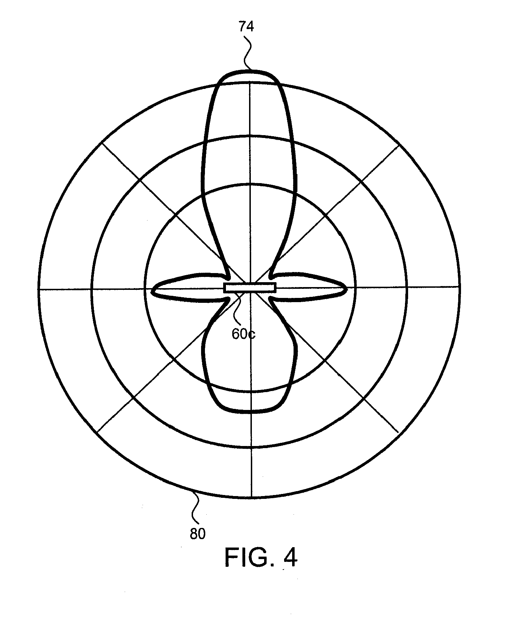

Fig. 4 zeigt ein schematisches Diagramm der Abstrahlcharakteristik eines 3λ/2-Dipols in einem Polardiagramm, gemäß einer ersten Ausführungsform der vorliegenden Erfindung.

Wie der Fig. 4 entnehmbar ist, weist die in einem Polarkoordinatensystem 80 aufgetragene Abstrahlcharakteristik eines 3λ/2-Dipols eine Keulencharakteristik 74 auf und hängt von der Bauform des Einzelstrahlers im Verhältnis zur Wellenlänge der abgestrahlten Strahlung ab und ihre Formung kann über eine Veränderung der Frequenz eingestellt werden. Im Fall des 3λ/2-Dipols 60c ist eine Richtwirkung der Abstrahlcharakteristik mit Abstrahlmaxima jeweils orthogonal und parallel zur Dipolachse gegeben.

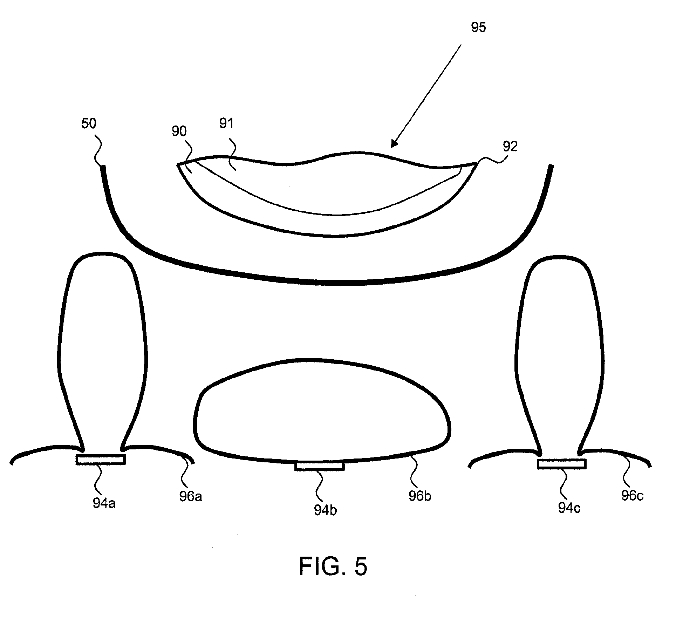

Fig. 5 zeigt eine schematische Darstellung eines menschlichen Gewebebereichs und seine räumliche Lage zu dem elektromagnetischen Gesamtfeld 50, welches von drei Dipolantennen der erfindungsgemäßen elektromedizinischen Vorrichtung erzeugt wird, gemäß einer ersten Ausführungsform der vorliegenden Erfindung.

Eine erste Dipolantenne 94a, eine zweite Dipolantenne 94b und eine dritte Dipolantenne 94c erzeugen durch die Überlagerung ihrer jeweilig unterschiedlichen Abstrahlcharakteristika 96a, 96b, 96c ein angepasstes elektromagnetisches Gesamtfeld 50. In der gezeigten Ausführungsform gemäß Fig. 5, wird dabei die zweite Dipolantenne 94b als λ-Dipol betrieben, während die erste Dipolantenne 94a und die dritte Dipolantenne 94c als 3λ/2-Dipol geschaltet werden. Die durch die erfindungsgemäße Anpassung erreichte Feldgeometrie kann dabei vorteilhafterweise durch den Umriss des zu behandelnden menschlichen Gewebebereichs 95 vorgegeben sein. Es versteht sich dabei, dass je nach Gewebebereich 95 unterschiedliche Feldgeometrien durch unterschiedliche Kombinationen von zu verschiedenen Antennen ausgestalteten Einzelstrahlern möglich sind.

Wie aus Fig. 5 ableitbar, ist ferner die Leistung der Dipolantennen 94a, 94b, 94c an eine gewünschten Eindringtiefe des elektromagnetisches Gesamtfeldes 50 in das Fettgewebe 90 angepasst. Dabei dringt das elektromagnetische Gesamtfeld 50 durch die Epidermis 92 ohne dabei zu tief in das Muskelgewebe 91 vorzudringen.

### Bezugszeichenliste

- elektromedizinische Vorrichtung: 5
- Basisteil: 6
- Erweiterungsteil: 8
- Richtwirkungs-Steuerung: 10
- Energiequelle: 11
- Steuereinrichtung: 14, 18, 22, 26
- Steuerglied: 12, 16, 20, 24
- erster Einzelstrahler: 32
- zweiter Einzelstrahler: 34
- dritter Einzelstrahler: 36
- vierter Einzelstrahler: 38
- elektromagnetisches Feld: 42, 44, 46, 48
- elektromagnetisches Gesamtfeld: 50
- λ/2-Dipol: 60a
- λ-Dipol: 60b
- 3λ/2-Dipol: 60c
- Kugelcharakteristik: 70
- Achtercharakteristik: 72
- Keulencharakteristik: 74
- Polarkoordinatensystem: 80
- Fettgewebe: 90
- Muskelgewebe: 91
- Epidermis: 92
- erste Dipolantenne: 94a
- zweite Dipolantenne: 94b
- dritte Dipolantenne: 94c
- menschlicher Gewebebereich: 95
- erste Abstrahlcharakteristik: 96a
- zweite Abstrahlcharakteristik: 96b
- dritte Abstrahlcharakteristik: 96c

## Patentansprüche

1. Elektromedizinische Vorrichtung zum nicht-invasiven Reduzieren oder Entfernen von subkutanem Fettgewebe,
- mit einer Energiequelle (11), die einen hochfrequenten Wechselstrom bereitstellt,
- mit zumindest zwei Einzelstrahlern (32, 34, 36, 38), die von der Energiequelle (11) gespeist werden und die dazu ausgelegt sind, hochfrequente elektromagnetische Wellen in subkutanes Fettgewebe auszustrahlen, und
- mit einer Richtwirkungs-Steuerung (10), die mit den Einzelstrahlern (32, 34, 36, 38) gekoppelt ist und die die Einzelstrahler (32, 34, 36, 38) derart ansteuert, dass durch Richtungs- und Feldbündelung der von den jeweiligen Einzelstrahlern (32, 34, 36, 38) ausgesendeten hochfrequenten elektromagnetischen Wellen ein elektromagnetisches Gesamtfeld (50) mit einer gewünschten Feldgeometrie in dem subkutanen Fettgewebe erzeugbar ist.

2. Vorrichtung nach Anspruch 1, **dadurch gekennzeichnet, dass** die Einzelstrahler (32, 34, 36, 38) kaskadenförmig oder matrixförmig zueinander angeordnet sind.

3. Vorrichtung nach Anspruch 2, **dadurch gekennzeichnet, dass** die kaskadenförmig oder matrixförmig angeordneten Einzelstrahler (32, 34, 36, 38) eine derartige Kaskadierung bzw. Matrizierung aufweisen, dass die gewünschte Feldgeometrie erzeugbar ist.

4. Vorrichtung nach einem der vorstehenden Ansprüche, **dadurch gekennzeichnet, dass** mindestens einer der Einzelstrahler (32, 34, 36, 38) als Dipolantenne ausgebildet ist.

5. Vorrichtung nach Anspruch 4, **dadurch gekennzeichnet, dass** mindestens eine der Dipolantennen als λ/2-Dipol (60a) oder λ-Dipol (60b) ausgebildet ist.

6. Vorrichtung nach einem der vorstehenden Ansprüche, **dadurch gekennzeichnet, dass** mindestens einer der Einzelstrahler (32, 34, 36, 38) als Punktstrahler ausgebildet ist.

7. Vorrichtung nach einem der vorstehenden Ansprüche, **dadurch gekennzeichnet, dass** die jeweiligen Einzelstrahler (32, 34, 36, 38) derart ausgerichtet sind, dass die Einzelstrahler (32, 34, 36, 38) die von ihnen erzeugten elektromagnetischen Wellen jeweils in dieselbe Richtung ausstrahlen.

8. Vorrichtung nach einem der vorstehenden Ansprüche, **dadurch gekennzeichnet, dass** für einen Einzelstrahler jeweils eine Steuereinrichtung (14, 18, 22, 26) vorgesehen ist, die dazu ausgelegt ist, die Frequenzen und/oder die Amplitude und/oder die Leistung der ausgestrahlten elektromagnetischen Wellen eines Einzelstrahlers (32, 34, 36, 38) zu verändern.

9. Vorrichtung nach Anspruch 8, **dadurch gekennzeichnet, dass** die Steuereinrichtung (14, 18, 22, 26) zumindest ein Laufzeitglied aufweist, wobei ein jeweiliges Laufzeitglied eingerichtet ist, das jeweilige elektromagnetische Feld (42, 44, 46, 48) eines Einzelstrahlers (32, 34, 36, 38) durch eine Änderung einer jeweiligen Signallaufzeit zu verändern.

10. Vorrichtung nach einem der vorstehenden Ansprüche, **dadurch gekennzeichnet, dass** für einen Einzelstrahler jeweils ein Steuerglied (12, 16, 20, 24) vorgesehen ist, welches zumindest einen Phasenschieber aufweist, wobei ein jeweiliger Phasenschieber dazu eingerichtet ist, das jeweilige elektromagnetische Feld (42, 44, 46, 48) der Einzelstrahler (32, 34, 36, 38) durch eine Änderung seiner jeweiligen Phase zu verändern.

11. Vorrichtung nach einem der vorstehenden Ansprüche, **dadurch gekennzeichnet, dass** die Vorrichtung dazu ausgelegt ist, im Frequenzbereich zwischen 0,4-61,5 GHz, vorzugsweise zwischen 1,0-7,5 GHz, besonders bevorzugt zwischen 1,6-5,9 GHz, und insbesondere zwischen 2,4-2,5 GHz zu arbeiten.
